# EUROPEAN PATENT APPLICATION

(11) **EP 2 939 600 A1**
(43) Date of publication of application: **04.11.2015**
(21) Application number: 13869428.6
(22) Date of filing: 01.11.2013
(51) Int. Cl.: A61B 8/08

(54) **ULTRASOUND DIAGNOSTIC DEVICE AND ELASTICITY ANALYSIS METHOD**

(30) Priority: 25.12.2012 JP 2012280423
(71) Applicant: Hitachi Aloka Medical, Ltd., Tokyo 181-8622 (JP)
(72) Inventor: TONOMURA, Akiko, Mitaka-shi Tokyo 181-8622 (JP)
(74) Representative: Strehl Schübel-Hopf & Partner
(86) International application number: PCT/JP2013/079749
(87) International publication number: WO 2014/103511

(57) **Abstract**

Provided are an ultrasound diagnostic device and an elasticity analysis method whereby it is possible to analyze a region in an elasticity image which is suited to analysis. An ultrasound diagnostic device comprises: a tomographic image construction unit (24) which constructs a tomographic image of a diagnostic site of a subject via an ultrasound probe; an elasticity information computation unit (34) which computes elasticity information which denotes hardness; an elasticity image construction unit (36) which constructs an elasticity image on the basis of the elasticity information which is computed in the elasticity information computation unit (34); an image display unit (28) which displays the tomographic image and the elasticity image; an analysis region detection unit (50) which detects an analysis region, wherein the elasticity information is analyzed, from distribution information of the elasticity information which constructs the elasticity image; and an analysis unit (52) which analyzes the elasticity information corresponding to the analysis region.

## Description

### TECHNICAL FIELD

The present invention generally relates to an ultrasound diagnostic device that displays a tomographic image and an elasticity image of the inside of a subject by the use of ultrasound waves and to an elasticity analysis method for analyzing the elasticity image.

### BACKGROUND ART

An ultrasound diagnostic device transmits ultrasound waves into a subject through an ultrasound probe and receives reflected echo signals that correspond to the structure of a biological tissue from the inside of the subject to construct and display a tomographic image of the inside of the subject.

Such an ultrasound diagnostic device also calculates and determines elasticity information such as strain of the tissue and the elastic modulus at a plurality of measurement points in the tomographic site of the subject, and generates and displays an elasticity image of the tomographic site based on the elasticity information. It is also common to use a histogram that is based on a displacement as auxiliary information for analyzing the mobility with respect to the level of infiltration into the periphery of a tumor (see

Patent Document 1).

### CITATION LIST

### PATENT LITERATURE

PATENT DOCUMENT 1: International Publication No. WO 2007/046272

### SUMMARY OF INVENTION

### TECHNICAL PROBLEM

With the use of only a histogram based on a displacement used as auxiliary information for analysis, an unnecessary-for-analysis region may unintentionally be analyzed if, for example, a displacement included in the unnecessary-for-analysis region, which is unsuitable for analysis because of inclusion of an artifact, is within an analysis range of the histogram. This may reduce the accuracy of analysis.

It is therefore an advantage of the present invention to analyze a region suitable for analysis in an elasticity image.

### SOLUTION TO PROBLEM

To solve the above problem, an ultrasound diagnostic device according to the present invention includes: a tomographic image construction unit that constructs a tomographic image of a diagnostic site of a subject via an ultrasound probe; an elasticity information computation unit that computes elasticity information indicative of hardness; an elasticity image construction unit that constructs an elasticity image based on the elasticity information computed in the elasticity information computation unit; an image display unit that displays the tomographic image and the elasticity image; an analysis region detection unit that detects an analysis region, in which the elasticity information is analyzed, from distribution information on the elasticity information constituting the elasticity image; and an analysis unit that analyzes the elasticity information corresponding to the analysis region.

### ADVANTAGEOUS EFFECT OF INVENTION

According to the present invention, a region suitable for analysis in an elasticity image can be analyzed.

### BRIEF DESCRIPTION OF DRAWINGS

FIG. 1 is a block diagram illustrating a configuration of an ultrasound diagnostic device according to the present invention.
FIG. 2 is a block diagram illustrating configurations of an analysis region detection unit and an analysis unit according to the present invention.
FIG. 3 shows diagrams illustrating displayed forms on an image display unit according to the present invention.
FIG. 4A is a diagram illustrating an example of distribution information on elasticity information in the depth direction of a scanning line according to the present invention.
FIG. 4B is a diagram illustrating an example of distribution information on elasticity information in the depth direction of a scanning line according to the present invention.
FIG. 5A is a diagram illustrating a displayed form on the image display unit according to the present invention.
FIG. 5B is a diagram illustrating a displayed form on the image display unit according to the present invention.
FIG. 5C is a diagram illustrating a displayed form on the image display unit according to the present invention.
FIG. 5D is a diagram illustrating a displayed form on the image display unit according to the present invention.
FIG. 6 is a flow chart showing an operation according to the present invention.
FIG. 7A is a diagram for describing Embodiment 2 according to the present invention.
FIG. 7B is a diagram for describing Embodiment 2 according to the present invention.
FIG. 7C is a diagram for describing Embodiment 2 according to the present invention.
FIG. 7D is a diagram for describing Embodiment 2 according to the present invention.
FIG. 7E is a diagram for describing Embodiment 2 according to the present invention.
FIG. 8 shows diagrams for describing Embodiment 3 according to the present invention.
FIG. 9 is a diagram for describing Embodiment 4 according to the present invention.

### DESCRIPTION OF EMBODIMENTS

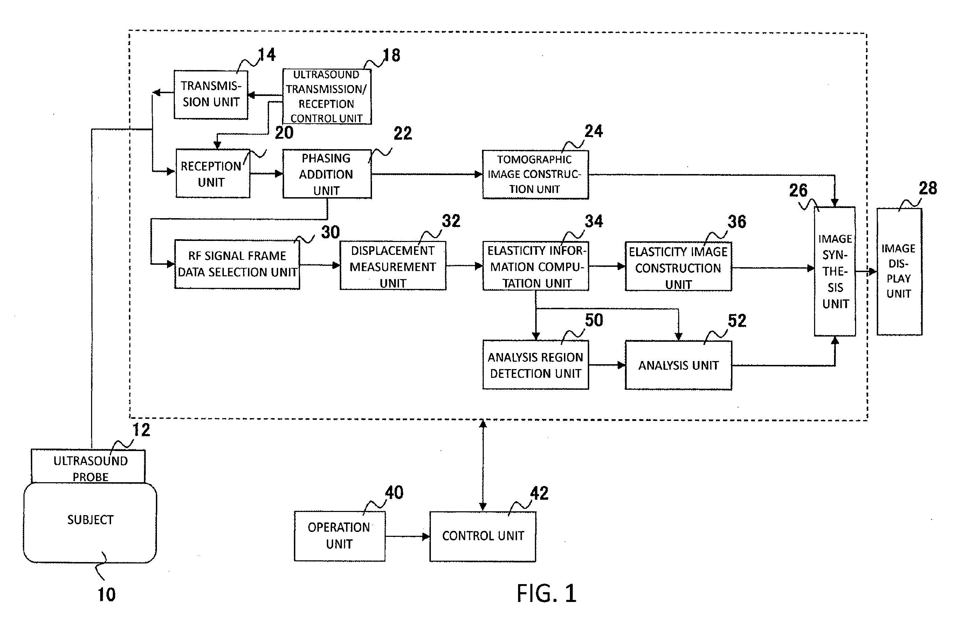

An ultrasound diagnostic device according to the present invention will now be described with reference to the drawings. FIG. 1 is a block diagram illustrating an ultrasound diagnostic device according to an embodiment of the present invention.

### EMBODIMENT 1

As shown in FIG. 1, an ultrasound diagnostic device according to the embodiment includes: an ultrasound probe 12 used in contact with a subject 10; a transmission unit 14 that repeatedly transmits ultrasound waves through the ultrasound probe 12 to the subject 10 with a certain time interval; a reception unit 20 that receives time-series reflected echo signals generated from the subject 10; an ultrasound transmission/reception control unit 18 that controls the transmission unit 14 and the reception unit 20; a phasing addition unit 22 that performs phasing addition on the received reflected echoes to generate RF signal frame data in a time series manner; a tomographic image construction unit 24 that constructs a tomographic image based on the RF signal frame data generated by the phasing addition unit 22; an image synthesis unit 26 that synthesizes the tomographic image with another image, numerical information, or the like; an image display unit 28 that displays an image output from the image synthesis unit 26; an RF signal frame data selection unit 30 that selects at least two pieces of RF signal frame data; a displacement measurement unit 32 that measures a displacement of a biological tissue of the subject 10 by the use of the selected RF signal frame data; an elasticity information computation unit 34 that calculates and determines elasticity information such as strain, elastic modulus, and the like, from the displacement measured by the displacement measurement unit 32; an elasticity image construction unit 36 that constructs an elasticity image from the elasticity information computed in the elasticity information computation unit 34; an operation unit 40 for an operator to operate; a control unit 42 that controls each component in response to the operation of the operation unit 40; an analysis region detection unit 50 that detects an analysis region suitable for analysis in the elasticity image; and an analysis unit 52 that analyzes the analysis region detected by the analysis region detection unit 50. A dashed line shown in FIG. 1 represents the body of the ultrasound diagnostic device.

The ultrasound probe 12 is formed of a plurality of transducers disposed therein, and transmits and receives ultrasound waves to and from the subject 10 through the transducers. The transmission unit 14 drives the ultrasound probe 12 to generate echo sounder pulses for generating ultrasound waves, and sets a convergent point of transmitted ultrasound waves to a certain depth and repeatedly transmits the ultrasound waves through the ultrasound probe 12 to the subject 10 with a certain time interval. The reception unit 20 has a function to receive time-series reflected echo signals generated from the subject 10 through the ultrasound probe 12 and amplify the received reflected echo signals with a predetermined gain to generate an RF signal (echo receiver signal). The ultrasound transmission/reception control unit 18 controls the transmission unit 14 and the reception unit 20 to transmit and receive ultrasound waves to and from the subject 10 through the ultrasound probe 12. The phasing addition unit 22 performs phasing addition on the reflected echo signals received by the reception unit 20. At this time, the phasing addition unit 22 phase-controls the RF signal amplified in the reception unit 20 input thereto to generate ultrasound beams for one or more convergent points and generates RF signal frame data, which is ultrasound tomographic data, in a time series manner.

The tomographic image construction unit 24 receives data on the tomographic site of the subject 10 input thereto, specifically the RF signal frame data from the phasing addition unit 22, and subjects the data to signal processing such as gain correction, log compression, envelope detection, contour enhancement, and filtering to construct tomographic image data (for example, a monochrome gray scale tomographic image of the subject 10). Although not shown, the tomographic image construction unit 24 includes an A/D converter that converts the tomographic image data into a digital signal, a frame memory that stores converted multiple pieces of tomographic image data in a time series manner, and a controller. The tomographic image data of the inside of the subject 10 stored in the frame memory is acquired as one image and the acquired tomographic image data is read in television synchronization.

The RF signal frame data selection unit 30 stores the RF signal frame data output from the phasing addition unit 22 and selects at least two pieces (one set) of RF signal frame data from a group of the stored RF signal frame data. For example, the RF signal frame data selection unit 30 sequentially stores the RF signal frame data generated in a time series manner; i.e., on the basis of the frame rates of the image, from the phasing addition unit 22, and selects a stored RF signal frame data set (N) as first data while the RF signal frame data selection unit 30 selects one set of RF signal frame data (X) among a group of RF signal frame data sets (N-1, N-2, N-3 ... N-M) that have been stored in the past in terms of time. Note that the characters N, M, and X are index numbers assigned to the RF signal frame data sets and are natural numbers.

The displacement measurement unit 32 measures a displacement of a biological tissue of the subject 10. Specifically, the displacement measurement unit 32 subjects the group of data sets; i.e. the RF signal frame data set (N) and the RF signal frame data set (X), selected by the RF signal frame data selection unit 30 to one or two dimensional correlation to calculate a vector representing displacement of a biological tissue corresponding to each measurement point in the tomographic image; i.e., one or two dimensional distribution of displacement relating to the direction and amount of the displacement. Here, the block matching or phase gradient method is used to detect the vector. In the block matching method, an image is divided into blocks of, for example, NxN pixels, and a block in the predetermined region (for example, a parameter acquisition region to be described later) is focused on. The previous frame is searched for a block that is most similar to the focused block in the current frame, and predictive coding, which is a process of determining a sampled value based on a difference, is performed with reference to the found frame. A displacement of each measurement point in the tomographic image is thereby calculated to detect the vector. In the phase gradient method, the amount of movement of a wave of a received signal is calculated from phase information of the wave so as to calculate a displacement of each measurement point in the tomographic image to detect the vector.

A pressure measurement unit, which is not shown, measures a stress in the subject 10 at a measurement point based on a pressure detected by a pressure sensor or the like provided between an ultrasound transmission/reception surface of the ultrasound probe 12 and the subject 10.

The elasticity information computation unit 34 calculates and determines elasticity information on the tissue at the tomographic site. The elasticity information refers to information such as strain, elastic modulus, and viscosity. In the embodiment, the elasticity information computation unit 34 calculates a strain and/or an elastic modulus of a biological tissue corresponding to each measurement point on the tomographic image based on displacement information on the biological tissue; for example, a motion vector, measured by the displacement measurement unit 32 by the use of RF signal frame data selected by the RF signal frame data selection unit 30. When the elasticity information computation unit 34 calculates an elastic modulus of a biological tissue, the unit takes into consideration a pressure value output from the pressure measurement unit. At this time, the data of strain is calculated by spatially differentiating the amount of movement; for example, a displacement of the biological tissue. The data of elastic modulus is calculated by dividing a change in pressure by a change in strain. For example, where a displacement measured by the displacement measurement unit 32 is L(X) and a pressure measured by the pressure measurement unit is P(X), the strain ΔS(X) can be calculated by spatially differentiating L(X), and then can be calculated by the equation ΔS(X) = ΔL(X)/ΔX. The Young's modulus Ym(X) of the elastic modulus data can be calculated by the equation Ym = ΔP(X)/ΔS(X). The elastic modulus of the biological tissue corresponding to each measurement point in the tomographic image can be calculated from the Young's modulus Ym, so that two dimensional elasticity image data can be obtained continuously. Note that Young's modulus refers to the ratio of a simple tensile stress applied to an object to a strain generated in parallel to the tension.

The elasticity image construction unit 36 constructs an elasticity image at a tomographic site based on elasticity information calculated and determined by the elasticity information computation unit 34. The elasticity image construction unit 36 includes a frame memory and an image processing unit, stores elasticity image frame data in the frame memory, and subjects the stored elasticity image frame data to image processing. The elasticity image construction unit 36 also has a function of providing hue information to the elasticity image frame data, and, based on the elasticity image frame data, converts it into image data that is given in terms of red (R), green (G), and blue (B), which are three primary colors. For example, the elasticity image construction unit 36 converts elasticity data with a large strain into a red code, and elasticity image data with small strain into a blue code.

The analysis region detection unit 50 first detects an unnecessary-for-analysis region that is unsuitable for analysis in an elasticity image. An unnecessary-for-analysis region refers to a region where, for example, there is an artifact based on elasticity information calculated and determined by the elasticity information computation unit 34. The analysis region detection unit 50 detects an unnecessary-for-analysis region where there is an artifact from distribution information on elasticity information. The analysis region detection unit 50 then detects an analyzable region from the remaining region other than the unnecessary-for-analysis region where there is an artifact and outputs it to the analysis unit 52 as an analysis region suitable for analysis in the elasticity image.

The analysis unit 52 analyzes the elasticity information for the analysis region detected by the analysis region detection unit 50. Analyzed information after analysis by the analysis unit 52 is displayed on the image display unit 28 through the image synthesis unit 26.

The image synthesis unit 26 is controlled by the control unit 42 based on image display conditions set via the operation unit 40. The operation unit 40 is provided with an operation device such as a mouse, keyboard, trackball, touch pen, or joystick so as to be able to set image display conditions and the like.

The image display unit 28 displays a tomographic image or an elasticity image as selected by an image selection unit in the image synthesis unit 26, as well as a composite image of the tomographic image and the elasticity image.

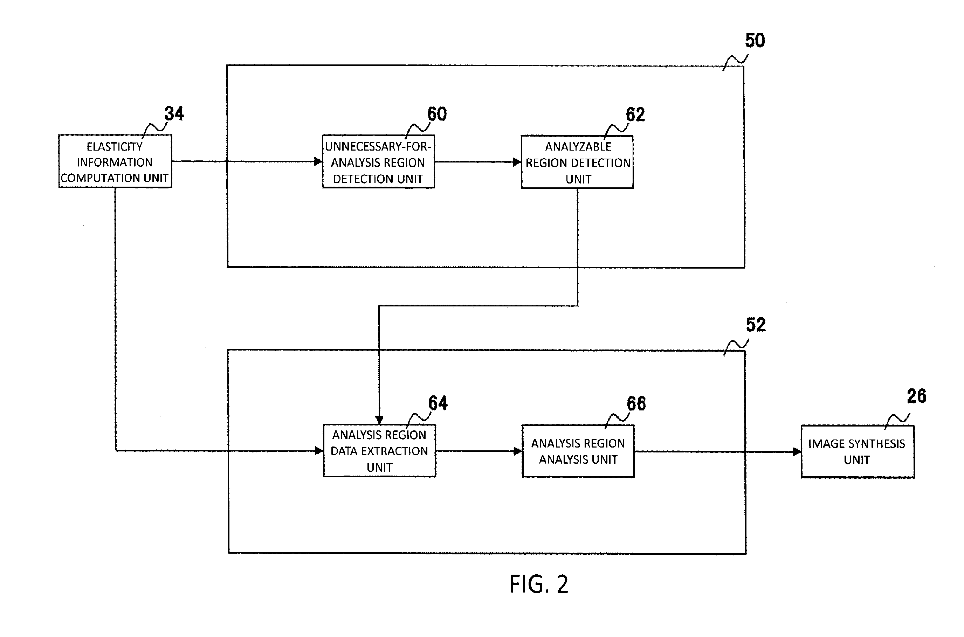

The configuration of the analysis region detection unit 50 and the analysis unit 52 will now be described with reference to FIG. 2. The analysis region detection unit 50 has an unnecessary-for-analysis region detection unit 60 that detects an unnecessary-for-analysis region that is unsuitable for analysis in an elasticity image, and an analyzable region detection unit 62 that detects an analyzable region from the remaining region other than the unnecessary-for-analysis region. The analysis unit 52 has an analysis region data extraction unit 64 that extracts elasticity information computed by the elasticity information computation unit 34 in an analysis region detected by the analyzable region detection unit 62, and an analysis region analysis unit 66 that analyzes the elasticity information in the analysis region.

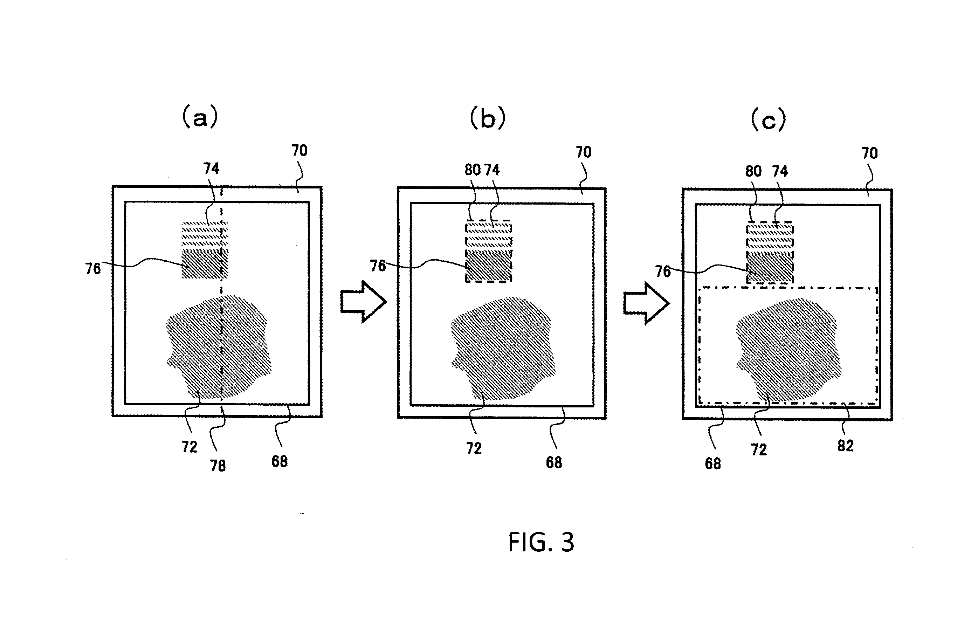

The analysis region detection unit 50 (the unnecessary-for-analysis region detection unit 60 and the analyzable region detection unit 62) will now be described specifically with reference to FIGS. 3 to 5. FIG. 3 is a composite image of a tomographic image 70 and an elasticity image 68 displayed on the image display unit 28. The tomographic image 70 is displayed so as to be consistent with a display area of the image display unit 28. The elasticity image 68 images the hardness of a tissue and is a region where the operator wishes to analyze, and is displayed so as to be consistent with a region set by the operator. A display area of the elasticity image 68 is included in the display area of the tomographic image 70.

As shown in FIG. 3(a), in the composite image of the tomographic image 70 and the elasticity image 68, a hard region 74 and a soft region 76 in the elasticity image 68 are displayed superposed on a tissue 72 in the tomographic image 70.

As shown in FIG. 3(a), when the hard region 74 and the soft region 76 are adjacent to each other in the depth direction, there may possibly be a cyst tissue or a multiple reflection region. A cyst is a tissue that is highly mobile and has no bonding between tissues. A multiple reflection region is a region where ultrasound waves are reflected in multiple ways, being in a fringe pattern when displayed. Such regions are displayed as an artifact in which the hard region 74 and the soft region 76 are adjacent to each other in the depth direction. Accordingly, as shown in FIG. 3(b), the unnecessary-for-analysis region detection unit 60 considers a region where the hard region 74 and the soft region 76 are adjacent to each other in the depth direction in the elasticity image 68 to be an artifact, and detects it as an unnecessary-for-analysis region that is unsuitable for analysis in the elasticity image 68.

When the hard region 74 and the soft region 76 are adjacent to each other in the depth direction in the elasticity image 68, elasticity information pulsates in the depth direction. Accordingly, the unnecessary-for-analysis region detection unit 60 detects a range of a scanning line in which the elasticity information pulsates in the depth direction as an unnecessary-for-analysis depth range. In this way, the unnecessary-for-analysis region detection unit 60 detects an unnecessary-for-analysis depth range in each scanning line constituting the elasticity image. The unnecessary-for-analysis region detection unit 60 then arranges unnecessary-for-analysis depth ranges of the respective scanning lines in the scanning direction and detects a collection of the unnecessary-for-analysis depth ranges (two dimensional region) as an unnecessary-for-analysis region. Note that the unnecessary-for-analysis region detection unit 60 may fail to detect an unnecessary-for-analysis depth range in a scanning line that does not constitute the elasticity image, because of the absence of elasticity information.

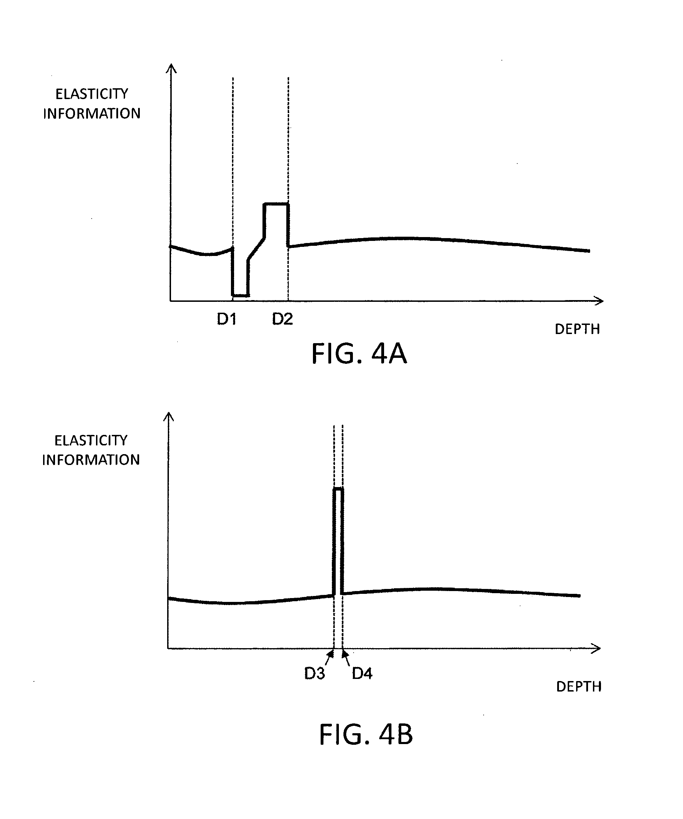

FIG. 4A illustrates an example of distribution information on elasticity information in the depth direction of a scanning line 78 shown in FIG. 3(a). As shown in FIG. 4A, the elasticity information pulsates in a depth range from depth D1 to depth D2. The unnecessary-for-analysis region detection unit 60 detects the depth range from depth D1 to depth D2 as an unnecessary-for-analysis region. The unnecessary-for-analysis region detection unit 60 then causes the scanning line 78 to shift in the scanning direction to detect an unnecessary-for-analysis depth range in each scanning line. The unnecessary-for-analysis region detection unit 60 arranges the unnecessary-for-analysis depth ranges in parallel in the scanning direction to detect an unnecessary-for-analysis region.

In some cases, spiked elasticity information may be detected in the depth direction and displayed as an artifact. Accordingly, the unnecessary-for-analysis region detection unit 60 detects a range in which elasticity information fluctuates abruptly in the depth direction (a spiked range) as an unnecessary-for-analysis depth range of the scanning line. The unnecessary-for-analysis region detection unit 60 detects an unnecessary-for-analysis depth range in each scanning line constituting the elasticity image. The unnecessary-for-analysis region detection unit 60 then arranges unnecessary-for-analysis depth ranges of the respective scanning lines in the scanning direction and detects a collection of the unnecessary-for-analysis depth ranges (two dimensional region) as an unnecessary-for-analysis region.

FIG. 4B illustrates an example of distribution information on elasticity information in the depth direction of a scanning line. As shown in FIG. 4B, elasticity information fluctuates abruptly into a spike shape in the depth direction in a depth range from depth D3 to depth D4. The unnecessary-for-analysis region detection unit 60 detects a depth range in which the wave height of elasticity information exceeds a predetermined threshold as an unnecessary-for-analysis depth range. The unnecessary-for-analysis region detection unit 60 may alternatively detect a depth range in which the slope of elasticity information exceeds a predetermined threshold as an unnecessary-for-analysis depth range. In this way, the unnecessary-for-analysis region detection unit 60 detects the depth range from depth D3 to depth D4 as an unnecessary-for-analysis depth range. The unnecessary-for-analysis region detection unit 60 then causes the scanning line 78 to shift in the scanning direction and arranges the unnecessary-for-analysis depth ranges of the respective scanning lines in parallel in the scanning direction to detect an unnecessary-for-analysis region.

As shown in FIG. 3(c), the analyzable region detection unit 62 then detects an analyzable region 82 from the remaining region other than an unnecessary-for-analysis region 80 detected by the unnecessary-for-analysis region detection unit 60. The analyzable region detection unit 62 detects the analyzable region 82 so that the unnecessary-for-analysis region 80 detected by the unnecessary-for-analysis region detection unit 60 is not included and the analyzable region 82 is within the display area of the elasticity image 68.

Specifically, assuming that the depth range of a unnecessary-for-analysis region 80 is from depth D1 to depth D2 and depth D1 (upper end) < depth D2 (lower end), the analyzable region detection unit 62 detects a range that is shallower than depth D1 of the unnecessary-for-analysis region 80 or a range that is deeper than depth D2 of the unnecessary-for-analysis region 80 as an analyzable region 82. FIG. 3(c) shows a form in which a range that is deeper than depth D2 of the unnecessary-for-analysis region 80 is detected as an analyzable region 82 by the analyzable region detection unit 62.

In this way, the analyzable region detection unit 62 detects the analyzable region 82 so that a scanning line that is in a range deeper than depth D2 of the unnecessary-for-analysis region 80 and is related to the elasticity image (an elasticity image-related scanning range) is included. A scanning range refers to a range in the scanning direction orthogonal to the depth direction. In other words, the analyzable region detection unit 62 detects an analyzable region 82 so that a scanning line that is in a range deeper than depth D2 of the unnecessary-for-analysis region 80 and has elasticity information that is present (a scanning range in which there is elasticity information, a scanning range for elasticity image 68) is included.

When the analyzable region detection unit 62 detects a range that is shallower than depth D1 of the unnecessary-for-analysis region 80 as the analyzable region 82, there is no tissue to be analyzed on the skin of the subject. Moreover, such a shallow range is generally a multiple reflection region. The analyzable region detection unit 62 therefore detects an analyzable region 82 excluding the skin of the subject or the range corresponding to the multiple reflection region. In other words, the analyzable region detection unit 62 detects an analyzable region 82 excluding a region that is shallower than a predetermined depth. For example, the analyzable region detection unit 62 detects as an analyzable region 82 a range that is deeper than a predetermined depth (for example, 1 cm) and shallower than depth D1 of the unnecessary-for-analysis region 80. Note that the operator can set such a predetermined depth to the analyzable region detection unit 62 via the operation unit 40.

Furthermore, assuming that the scanning range of the unnecessary-for-analysis region 80 is from scanning position S1 to scanning position S2 and the scanning position S1 is at the left end and the scanning position S2 is at the right end, the analyzable region detection unit 62 detects a range on the left side from the scanning position S1 of the unnecessary-for-analysis region 80 or a range on the right side from the scanning position S2 of the unnecessary-for-analysis region 80 as the analyzable region 82.

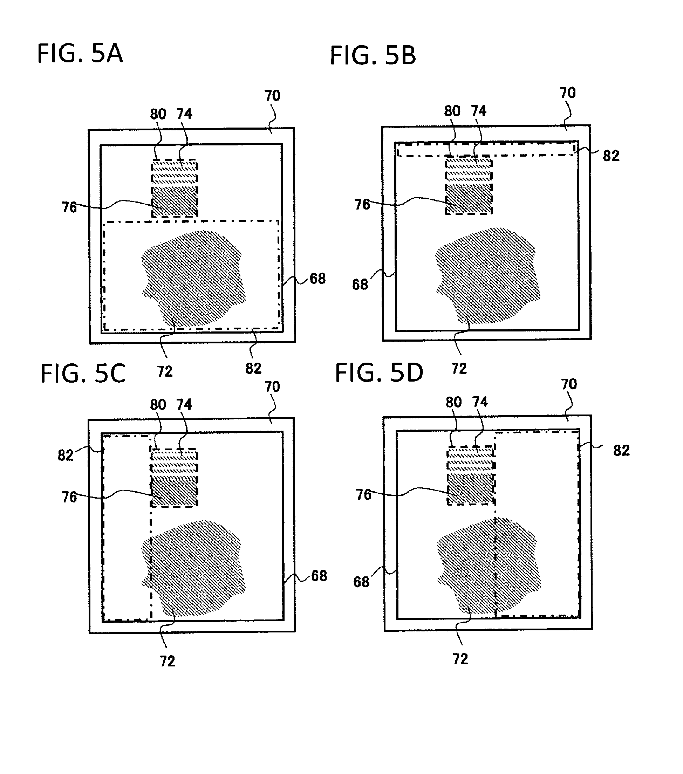

FIGS. 5A to 5D illustrate a plurality of forms of analyzable region 82 detected by the analyzable region detection unit 62. FIG. 5A shows a form in which an analyzable region 82 in a range deeper than the lower end of the unnecessary-for-analysis region 80 is detected. FIG. 5B shows a form in which an analyzable region 82 in a range shallower than the upper end of the unnecessary-for-analysis region 80 is detected. FIG. 5C shows a form in which an analyzable region 82 on the left side from the left end of the unnecessary-for-analysis region 80 is detected. FIG. 5D shows a form in which an analyzable region 82 on the right side from the right end of the unnecessary-for-analysis region 80 is detected. These analyzable regions 82 are all within the display area of the elasticity image 68.

As shown in FIGS. 5A to 5D, a plurality of analyzable regions 82 detected by the analyzable region detection unit 62 are displayed on the image display unit 28. The operator can select one analyzable region 82 among the plurality of analyzable regions 82 detected by the analyzable region detection unit 62 via the operation unit 40. The control unit 42 receives an indication of selection made on the operation unit 40 and communicates the selected one of the analyzable regions 82 to the analyzable region detection unit 62. The selected analyzable region 82 is output to the analysis unit 52 as an analysis region detected by the analysis region detection unit 50 (analyzable region detection unit 62).

Alternatively, the analyzable region detection unit 62 may automatically select the analyzable region 82 that has the largest area (largest analyzable region) among the plurality of analyzable regions 82 displayed as shown in FIGS. 5A to 5D. The selected analyzable region 82 is output to the analysis unit 52 with the largest analyzable region detected by the analysis region detection unit 50 (analyzable region detection unit 62) serving as the analysis region.

The analysis region data extraction unit 64 extracts elasticity information computed by the elasticity information computation unit 34 at coordinates corresponding to an analysis region detected by the analyzable region detection unit 62. For example, when the analyzable region 82 shown in FIG. 5A is detected as the analysis region, the analysis region data extraction unit 64 extracts elasticity information at coordinates corresponding to the analyzable region 82 shown in FIG. 5A. When the analyzable region 82 shown in FIG. 5B is detected as the analysis region, the analysis region data extraction unit 64 extracts elasticity information at coordinates corresponding to the analyzable region 82 shown in FIG. 5B. Similarly, when the analyzable region 82 that has the largest area (largest analyzable region) is automatically selected, the analysis region data extraction unit 64 extracts elasticity information at coordinates corresponding to the largest analyzable region.

The analysis region analysis unit 66 analyzes elasticity information extracted by the analysis region data extraction unit 64. In other words, the analysis region analysis unit 66 analyzes elasticity information in an analysis region. Specifically, the analysis region analysis unit 66 counts the number of appearance of each piece of elasticity information in an analysis region and creates histogram data for the analysis region. Based on the histogram data, the analysis region analysis unit 66 then calculates statistic values such as average, median, mode value, variance, standard deviation, quartile, maximum, and minimum.

Alternatively, the analysis region analysis unit 66 may binarize an analysis region by a threshold and calculate complexity. When the complexity calculated by the analysis region analysis unit 66 is high, the operator can recognize that the tissue has a structure of complex shape; i.e., a structure of a mottled pattern. When the complexity is low, the operator can recognize that the tissue has a circular structure. The result of analysis performed in the analysis region analysis unit 66 is displayed on the image display unit 28.

Although the analysis unit 52 performs analysis excluding the unnecessary-for-analysis region 80 that is unsuitable for analysis because of inclusion of an artifact in the elasticity image 68, the image display unit 28 displays as an artifact the elasticity image 68 of the region corresponding to the unnecessary-for-analysis region 80 as it is. In other words, the display area of the elasticity image 68 remains unchanged. Accordingly, the operator can verify the analysis range and the analysis status of the analysis region while verifying the elasticity image 68 corresponding to the unnecessary-for-analysis region 80.

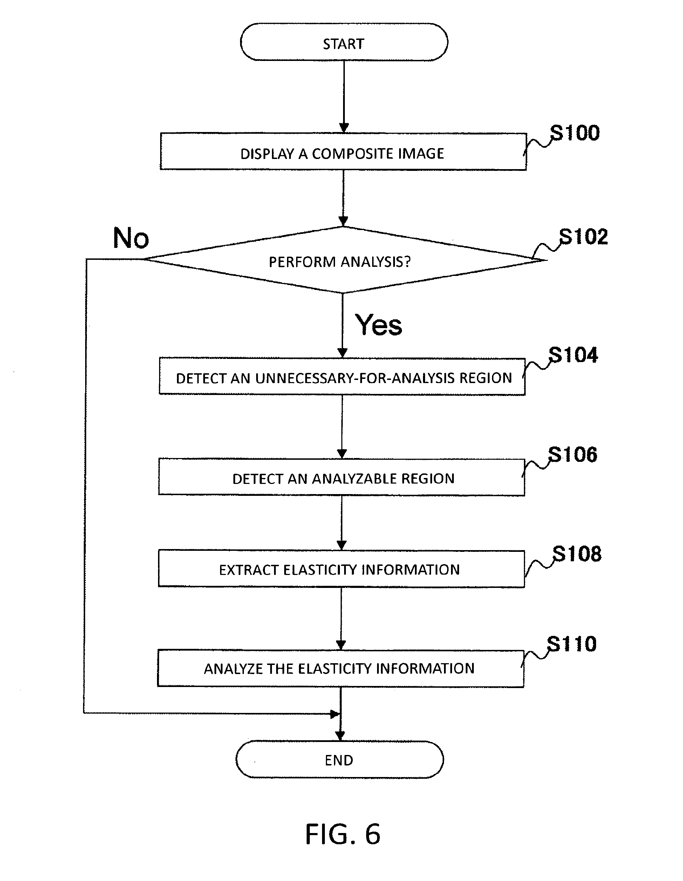

The operation of the embodiment will now be described with reference to FIG. 6.

(S100) The image display unit 28 displays a composite image of a tomographic image 70 constructed by the tomographic image construction unit 24 and an elasticity image 68 based on elasticity information constructed by the elasticity image construction unit 36. The operator sets the display area of the elasticity image 68 so as to include a region where the operator wants to analyze by means of the analysis unit 52 and views the elasticity image.

(S102) When the intention is to analyze the elasticity information, the process proceeds to S104. When the intention is not to analyze the elasticity information, the process ends.

(S104) the analysis region detection unit 50 detects an unnecessary-for-analysis region that is unsuitable for analysis (a region corresponding to an artifact) in the elasticity image.

(S106) the analysis region detection unit 50 detects an analyzable region (a region suitable for analysis) from the remaining region other than the unnecessary-for-analysis region. Accordingly, a region suitable for analysis can be detected in the region where the operator wants to analyze by means of the analysis unit 52.

(S108) The analysis unit 52 extracts elasticity information computed by the elasticity information computation unit 34 in the detected analysis region. Accordingly, elasticity information in the analysis region suitable for analysis is extracted.

(S110) The analysis unit 52 analyzes the elasticity information in the analysis region suitable for analysis.

As described above, the ultrasound diagnostic device according to the embodiment includes: the tomographic image construction unit 24 that constructs a tomographic image of a diagnostic site of a subject via an ultrasound probe; the elasticity information computation unit 34 that computes elasticity information indicative of hardness; the elasticity image construction unit 36 that constructs an elasticity image based on the elasticity information computed in the elasticity information computation unit 34; the image display unit 28 that displays the tomographic image and the elasticity image; the analysis region detection unit 50 that detects an analysis region, in which the elasticity information is analyzed, from distribution information on the elasticity information constituting the elasticity image; and the analysis unit 52 that analyzes the elasticity information corresponding to the analysis region. The ultrasound diagnostic device according to the embodiment further includes: the transmission unit 14 that transmits ultrasound waves through the ultrasound probe 12 to the subject; the reception unit 20 that receives the ultrasound waves reflected on the subject through the ultrasound probe 12; the elasticity information computation unit 34 that calculates and determines elasticity information at the tomographic site of the subject based on data generated from the ultrasound waves received in the reception unit 20; the analysis region detection unit 50 that detects an unnecessary-for-analysis region at the tomographic site based on the elasticity information; and the analysis unit 52 that analyzes the tomographic site for a region other than the unnecessary-for-analysis region based on the elasticity information. In the ultrasound diagnostic device according to the embodiment, the analysis region detection unit 50 includes the unnecessary-for-analysis region detection unit 60 that detects an unnecessary-for-analysis range in the depth direction corresponding to the direction in which direction ultrasound waves are transmitted and received, based on fluctuation of the elasticity information in the depth direction so as to detect the unnecessary-for-analysis range for each of a plurality of lines that extend in the depth direction and are arranged in the direction intersecting the depth direction and to detect an unnecessary-for-analysis region. In the ultrasound diagnostic device according to the embodiment, the analysis region detection unit 50 detects an unnecessary-for-analysis region based on the level of variation of the elasticity information at the tomographic site.

Furthermore, an elasticity analysis method includes: constructing a tomographic image of a diagnostic site of a subject; computing elasticity information indicative of hardness; constructing an elasticity image based on the elasticity information; displaying the tomographic image and the elasticity image; detecting an analysis region, in which the elasticity information is analyzed, from distribution information on the elasticity information constituting the elasticity image; and analyzing the elasticity information corresponding to the analysis region.

Accordingly, a region suitable for analysis in an elasticity image can be analyzed by excluding an unnecessary-for-analysis region that is unsuitable for analysis because of inclusion of an artifact in an elasticity image.

In the embodiment, although for the sake of simplicity a rectangular area is set as the analyzable region, it may be any of polygon, circle, ellipse, concave, convex, and the like, so long as the analyzable region is set so as to exclude the unnecessary-for-analysis region.

The analysis region detection unit 50 may detect an unnecessary-for-analysis region from blood flow information. For example, the analysis region detection unit 50 detects an unnecessary-for-analysis region from the presence or absence of blood flow. A region where a blood flow is present is a region where elasticity information cannot accurately be computed and therefore is an artifact.

### EMBODIMENT 2

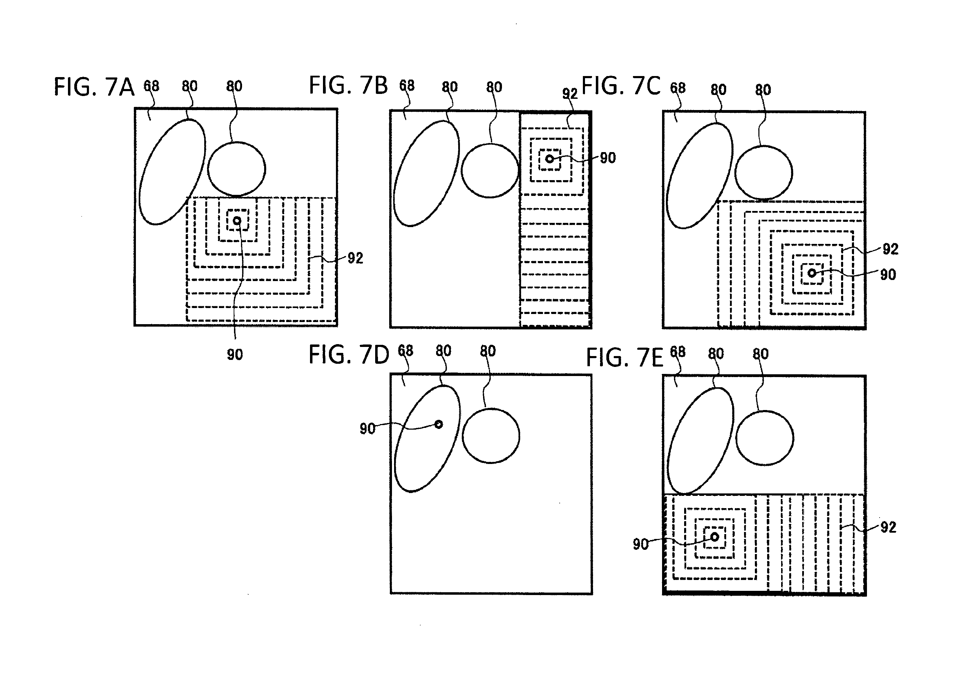

Embodiment 2 will now be described with reference to FIGS. 7A to 7E. The difference from Embodiment 1 is that the analyzable region detection unit 62 detects one analyzable region among a plurality of analyzable regions set based on a plurality of reference points.

The analyzable region detection unit 62 detects an analyzable region 82 from the remaining region other than an unnecessary-for-analysis region 80 detected by the unnecessary-for-analysis region detection unit 60 based on a plurality of reference points. The analyzable region detection unit 62 sets a plurality of reference points 90 on an elasticity image 68.

As shown in FIGS. 7A to 7E, for example, five reference points are set on the elasticity image 68 in a dispersed manner. In FIG. 7A, a reference point 90 is set at the center of the elasticity image 68. In FIG. 7B, a reference point 90 is set in the upper right of the elasticity image 68. In FIG. 7C, a reference point 90 is set in the lower right of the elasticity image 68. In FIG. 7D, a reference point 90 is set in the upper left of the elasticity image 68. In FIG. 7E, a reference point 90 is set in the lower left of the elasticity image 68.

The analyzable region detection unit 62 enlarges a rectangular region 92 with the set reference point 90 being at the center to detect an analyzable region. Specifically, the analyzable region detection unit 62 enlarges the rectangular region 92 with the set reference point 90 being at the center and when the enlarged rectangular region 92 overlaps with the unnecessary-for-analysis region 80 or overlaps with the display area of the elasticity image 68, the enlargement of the rectangular region 92 in the overlapping direction ends. The analyzable region detection unit 62 stores the outer periphery (perimeter) of the rectangular region 92 for which the enlargement ends as an analyzable region of the reference point 90. In this way, the analyzable regions for the number of reference points are stored in the analyzable region detection unit 62. In this case, five reference points are set, which means that five analyzable regions are stored in the analyzable region detection unit 62.

Taking FIG. 7A as an example, the rectangular region 92 is enlarged in four directions: upward, downward, leftward, and rightward. For the rightward and downward directions of the rectangular region 92, the enlarged rectangular region 92 overlaps with the display area of the elasticity image 68. Accordingly, the enlargement of the rectangular region 92 in the rightward direction ends at the right end of the display area of the elasticity image 68, and the enlargement of the rectangular region 92 in the downward direction ends at the lower end of the display area of the elasticity image 68. For the leftward and upward directions of the rectangular region 92, the enlarged rectangular region 92 overlaps with the unnecessary-for-analysis region 80. Accordingly, the enlargement of the rectangular region 92 in the leftward direction ends at the right end of the unnecessary-for-analysis region 80, and the enlargement of the rectangular region 92 in the upward direction ends at the lower end of the unnecessary-for-analysis region 80. Forms in FIGS. 7B, 7C, and 7E are similar to that in FIG. 7A.

Note that although the reference point 90 is set in the upper left of the elasticity image 68 in the form of FIG. 7D, the set reference point 90 is within the unnecessary-for-analysis region 80 and therefore the rectangular region 92 is not enlarged. Accordingly, no analyzable region is detected in the form of FIG. 7D. This means that an undetected analyzable region is not stored in the analyzable region detection unit 62. The analyzable region detection unit 62 then selects an analyzable region that has the largest area (largest analyzable region) among a plurality of stored analyzable regions. Accordingly, an analysis region suitable for analysis can be detected efficiently in the region where the operator wants to analyze by means of the analysis unit 52.

Note that the analyzable region detection unit 62 may detect one analyzable region by connecting (superimposing) a plurality of analyzable regions set based on a plurality of reference points. Specifically, as shown in the forms in FIGS. 7A to 7E, a plurality of rectangular regions 92 enlarged with a plurality of reference points 90 are detected. The analyzable region detection unit 62 stores the outer periphery (perimeter) of the enlarged rectangular regions 92 as an analyzable region of the reference point 90. The analyzable region detection unit 62 connects (superimposes) a plurality of stored analyzable regions to detect one analyzable region. In other words, the outer periphery

(perimeter) of the plurality of analyzable regions connected (superimposed) together is detected as one analyzable region.

The analysis unit 52 extracts elasticity information computed by the elasticity information computation unit 34 in the detected analysis region. Accordingly, elasticity information in a region suitable for analysis is extracted. The analysis unit 52 analyzes the elasticity information in the analysis region suitable for analysis.

As described above, according to the embodiment, the analyzable region detection unit 62 can efficiently detect the analyzable region 82 so that the unnecessary-for-analysis region 80 detected by the unnecessary-for-analysis region detection unit 60 is not included and the analyzable region 82 is within the display area of the elasticity image 68.

### EMBODIMENT 3

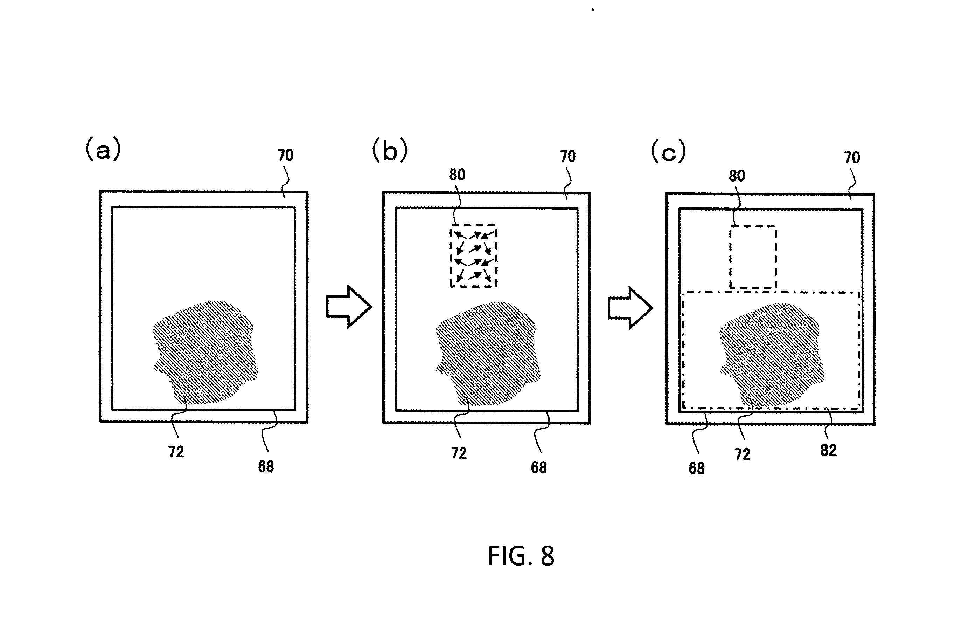

Embodiment 3 will now be described with reference to FIG. 8. The difference from Embodiments 1 and 2 is that the unnecessary-for-analysis region detection unit 60 uses a variation or a dispersion (the level of variation) of elasticity information to detect an unnecessary-for-analysis region. Elasticity information as used in the embodiment refers to elasticity information, such as displacement and strain, which has a directional property.

The unnecessary-for-analysis region detection unit 60 measures a variation for distribution information on elasticity information in a certain region relative to distribution information on elasticity information in surroundings, and detects a region with a large variation compared to the distribution information on elasticity information in surroundings as an unnecessary-for-analysis region. Alternatively, the unnecessary-for-analysis region detection unit 60 may calculate a dispersion of elasticity information in a certain region and detect as an unnecessary-for-analysis region a region with a calculated dispersion larger than a threshold.

As shown in FIG. 8(a), in the composite image of the tomographic image 70 and the elasticity image 68, a hard region 74 and a soft region 76 in the elasticity image 68 are displayed superposed on a tissue 72 in the tomographic image 70.

Then, as shown in FIG. 8(b), the unnecessary-for-analysis region detection unit 60 uses a dispersion of a variation of elasticity information to detect in the elasticity image 68 an unnecessary-for-analysis region 80 that is unsuitable for analysis. Arrows displayed in the unnecessary-for-analysis region 80 show the magnitude and direction of elasticity information at each measurement point. While the unnecessary-for-analysis region detection unit 60 detects an artifact described in Embodiment 1 as an unnecessary-for-analysis region 80, it may also use a dispersion or a variation to detect the unnecessary-for-analysis region 80.

As shown in FIG. 8(c), the analyzable region detection unit 62 then detects an analyzable region 82 from the remaining region other than the unnecessary-for-analysis region 80 detected by the unnecessary-for-analysis region detection unit 60. In other words, a region with a small variation or dispersion is detected as the analyzable region 82. The analyzable region detection unit 62 detects the analyzable region 82 so that the unnecessary-for-analysis region 80 detected by the unnecessary-for-analysis region detection unit 60 is not included and the analyzable region 82 is within the display area of the elasticity image 68.

As described above, according to the embodiment, since a variation or a dispersion of elasticity information is used to detect an unnecessary-for-analysis region, the accuracy of analysis can be improved.

### EMBODIMENT 4

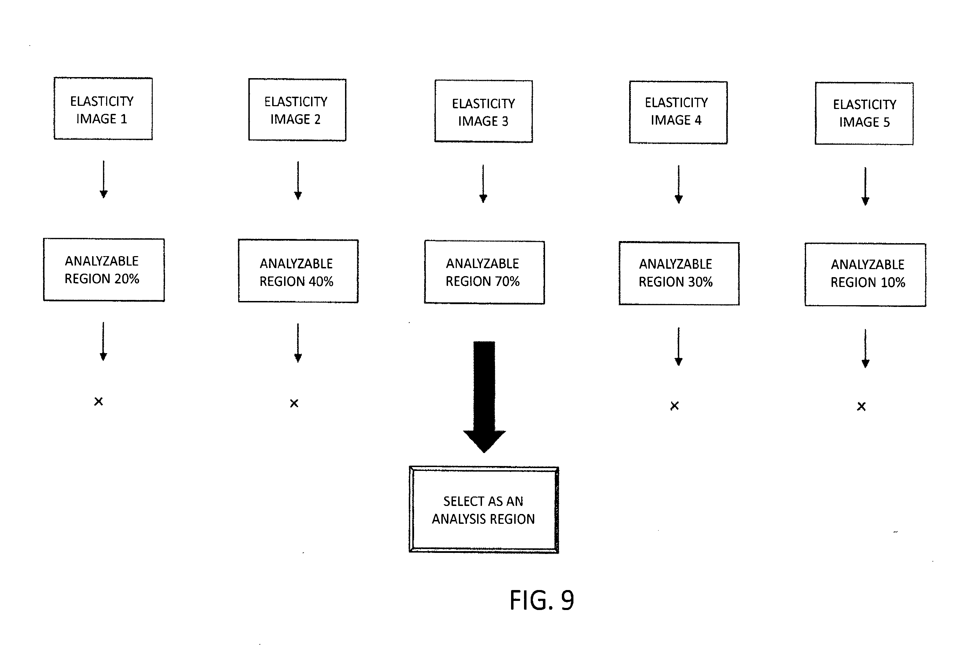

Embodiment 4 will now be described with reference to FIG. 9. The difference from Embodiments 1 to 3 is that the analysis unit 52 analyzes an elasticity image in which there is an analyzable region that has the largest area, among a plurality of elasticity images that are constructed by the elasticity image construction unit 36 and differ in time from each other.

It is assumed here that elasticity images 1 to 5 are constructed in a time series manner by the elasticity image construction unit 36. The percentage of an elasticity image is that of the analyzable region in relation to the display area. For example, for the elasticity image 1, the percentage of the analyzable region to the display area of the elasticity image is 20%. For the elasticity image 3, the percentage of the analyzable region to the display area of the elasticity image is 70%. In the elasticity images 1 to 5, the percentage of the analyzable region of the elasticity image 3 is the largest.

The analysis unit 52 selects the elasticity image 3 in which there is an analyzable region that has the largest area and analyzes as the analysis region the analyzable region that has the largest area. Accordingly, a larger region can be analyzed for the analysis region where the operator wishes to analyze.

Alternatively, the analysis unit 52 may select an elasticity image that is at or above a predetermined percentage for analysis. Since an elasticity image that is below a predetermined percentage is not selected, the analysis accuracy is improved. Note that any predetermined percentage can be set by the operator, by means of the operation unit 40.

### REFERENCE SIGNS LIST

10 subject; 12 ultrasound probe; 14 transmission unit; 18 ultrasound transmission/reception control unit; 20 reception unit; 22 phasing addition unit; 24 tomographic image construction unit; 26 image synthesis unit; 28 image display unit; 30 RF signal frame data selection unit; 32 displacement measurement unit; 34 elasticity information computation unit; 36 elasticity image construction unit; 40 operation unit; 42 control unit; 50 analysis region detection unit; 52 analysis unit

## Claims

1. An ultrasound diagnostic device comprising:
a tomographic image construction unit that constructs a tomographic image of a diagnostic site of a subject via an ultrasound probe;
an elasticity information computation unit that computes elasticity information indicative of hardness;
an elasticity image construction unit that constructs an elasticity image based on the elasticity information computed in the elasticity information computation unit;
an image display unit that displays the tomographic image and the elasticity image;
an analysis region detection unit that detects an analysis region, in which the elasticity information is analyzed, from distribution information on the elasticity information constituting the elasticity image; and
an analysis unit that analyzes the elasticity information corresponding to the analysis region.

2. The ultrasound diagnostic device according to claim 1, wherein
the analysis region detection unit detects an unnecessary-for-analysis region where there is an artifact from distribution information on elasticity information.

3. The ultrasound diagnostic device according to claim 2, wherein
the analysis region detection unit detects an analyzable region from the remaining region other than the unnecessary-for-analysis region where there is an artifact and output it to the analysis unit as the analysis region.

4. The ultrasound diagnostic device according to claim 1, wherein
the analysis region detection unit has an unnecessary-for-analysis region detection unit that detects an unnecessary-for-analysis region that is unsuitable for analysis in the elasticity image and an analyzable region detection unit that detects an analyzable region from the remaining region other than the unnecessary-for-analysis region.

5. The ultrasound diagnostic device according to claim 1, wherein
the analysis unit has an analysis region data extraction unit that extracts elasticity information computed by the elasticity information computation unit in the analysis region, and an analysis region analysis unit that analyzes the elasticity information in the analysis region.

6. The ultrasound diagnostic device according to claim 4, wherein
the unnecessary-for-analysis region detection unit detects a region where a hard region and a soft region are adjacent to each other in the depth direction as the unnecessary-for-analysis region.

7. The ultrasound diagnostic device according to claim 4, wherein
the unnecessary-for-analysis region detection unit detects an unnecessary-for-analysis depth range in each scanning line constituting the elasticity image, and arranges unnecessary-for-analysis depth ranges of the respective scanning lines in the scanning direction and detects a collection of the unnecessary-for-analysis depth ranges as the unnecessary-for-analysis region.

8. The ultrasound diagnostic device according to claim 4, wherein
the analyzable region detection unit detects the analyzable region so that an unnecessary-for-analysis region detected by the unnecessary-for-analysis region detection unit is not included and the analyzable region is within a display area of the elasticity image.

9. The ultrasound diagnostic device according to claim 4, wherein
the analyzable region detection unit selects the analyzable region that has the largest area among a plurality of selected analyzable regions.

10. The ultrasound diagnostic device according to claim 2, wherein
the image display unit displays as an artifact the elasticity image of a region corresponding to the unnecessary-for-analysis region.

11. The ultrasound diagnostic device according to claim 4, wherein
the analyzable region detection unit detects one analyzable region among a plurality of analyzable regions set based on a plurality of reference points.

12. The ultrasound diagnostic device according to claim 11, wherein
the analyzable region detection unit enlarges a rectangular region with the reference point being at center to detect the analyzable region.

13. The ultrasound diagnostic device according to claim 4, wherein
the unnecessary-for-analysis region detection unit uses a variation or a dispersion of the elasticity information to detect the unnecessary-for-analysis region.

14. The ultrasound diagnostic device according to claim 1, wherein
the analysis unit analyzes an elasticity image in which there is an analyzable region that has the largest area, among a plurality of elasticity images that are constructed by the elasticity image construction unit and differ in time from each other.

15. An elasticity analysis method comprising:
constructing a tomographic image of a diagnostic site of a subject;
computing elasticity information indicative of hardness;
constructing an elasticity image based on the elasticity information;
displaying the tomographic image and the elasticity image;
detecting an analysis region, in which the elasticity information is analyzed, from distribution information on the elasticity information constituting the elasticity image; and
analyzing the elasticity information corresponding to the analysis region.
